# EUROPÄISCHE PATENTSCHRIFT

(11) **EP 0 846 095 B1**
(45) Veröffentlichungstag und Bekanntmachung des Hinweises auf die Patenterteilung: **25.10.2000**
(21) Anmeldenummer: 96929290.3
(22) Anmeldetag: 21.08.1996
(51) Int. Cl.: C07C 45/50

(54) **VERFAHREN ZUR AUFARBEITUNG EINES FLÜSSIGEN HYDROFORMYLIERUNGSAUSTRAGS**
PROCESS FOR RECYCLING A LIQUID HYDROFORMYLATION DISCHARGE
PROCEDE DE RECYCLAGE DU PRODUIT LIQUIDE DE DECHARGE D'UN PROCESSUS D'HYDROFORMYLATION

(30) Priorität: 21.08.1995 DE 19530698
(43) Veröffentlichungstag der Anmeldung: 10.06.1998
(73) Patentinhaber: BASF AKTIENGESELLSCHAFT, 67056 Ludwigshafen (DE)
(72) Erfinder: BORGEL, Franz, D-67246 Dirmstein (DE); MÜLLER, Rolf, D-67125 Dannstadt-Schauernheim (DE); KROKOSZINSKI, Roland, D-67273 Weisenheim (DE)
(86) Internationale Anmeldenummer: EP9603665
(87) Internationale Veröffentlichungsnummer: WO9707086

(56) Entgegenhaltungen:
- EP-A- 0 404 193
- US-A- 3 455 091

## Beschreibung

Die vorliegende Erfindung betrifft ein Verfahren zur Aufarbeitung eines im wesentlichen flüssigen, Aldehyde und nicht umgesetzte Olefine enthaltenden Austrags aus einem Hydroformylierungsreaktor aus der mit Hilfe eines homogen im Reaktionsmedium gelösten phosphorhaltigen Rhodium-Katalysators katalysierten Niederdruckhydroformylierung bei 50 bis 150°C und 2 bis 30 bar von C₂- bis C₂₀-olefinen oder Olefingemischen, die verschiedene Isomere der betreffenden Olefine enthalten.

Die Hydroformylierung von Olefinen zu den entsprechenden Aldehyden ist von enormer wirtschaftlicher Bedeutung, da die auf diesem Wege hergestellten Aldehyde wiederum Ausgangsmaterial für eine Vielzahl großtechnischer Produkte, wie Lösungsmittel oder Weichmacheralkohole sind. Dementsprechend werden Hydroformylierungsverfahren weltweit stark beforscht, um weitere wirtschaftliche Verbesserungen, beispielsweise bei der Energiebilanz des Verfahrens, zur Erhöhung von dessen Selektivität und zur Schonung des homogenen Rhodiumkatalysators zu erzielen.

Für die Hydroformylierung von C₂- bis C₂₀-olefinen zu den entsprechenden Aldehyden wird im allgemeinen das sogenannten Flüssigaustragsverfahren (US-A 4 148 830, EP-A 16286) angewandt, bei dem der im wesentlichen, bis auf das im Überschuß zur Hydroformylierung eingesetzte Synthesegas, flüssige Austrag aus dem Hydroformylierungsreaktor in ein Entspannungsgefäß entspannt wird, wobei, infolge der Druckerniedrigung, der Austrag in eine flüssige, den Katalysator, Lösungsmittel, hochsiedende Nebenprodukte und geringere Menge an Aldehyd und nicht umgesetztem Olefin enthaltende flüssige Phase und eine neben dem überschüssigen Synthesegas den Hauptteil des gebildeten Aldehyds und des nicht umgesetzten Olefins enthaltende Gasphase aufgetrennt wird. Die flüssige Phase wird dann als Rückführstrom wieder in den Reaktor geleitet und die Gasphase abgezogen, das Synthesegas daraus abgetrennt und anschließend destillativ in Aldehyd und Olefine aufgetrennt. Der flüssige Rückführstrom enthält, verglichen mit dem aus dem Entspannungsbehälter abgezogenen Gasstrom, zwar geringe, aber für die Wirtschaftlichkeit des Verfahrens nicht vernachlässigbare Mengen an Aldehyd und Olefin. Abgesehen davon, daß es unwirtschaftlich ist, auf diese Weise ständig eine bestimmte Aldehydmenge im Kreis zu fahren, neigt der rückgeführte Aldehyd unter den Hydroformylierungsbedingungen zur Bildung hochsiedender Kondensationsprodukte. Letztere sind zwar ein gutes Lösungsmittel für die Hydroformylierung, doch ist man aus Gründen der Wirtschaftlichkeit bestrebt, die Neubildung solch hochsiedender Nebenprodukte in engen Grenzen zu halten. Nachteilig kann auch die Rückführung von im flüssigen Rückführstrom gelösten Restmengen an Olefin sein, und zwar dann, wenn als Ausgangsstoff für die Hydroformylierung nicht ein reines Olefin verwendet wird, sondern ein neben dem umzusetzenden Olefin noch andere isomere Olefine und gesättigte Kohlenwasserstoffe enthaltendes Kohlenwasserstoffgemisch. Hierbei kann es zur unerwünschten Aufpegelung solcher Olefine, die wiederum die Bildung unerwünschter Nebenprodukte zur Folge haben kann, und im weiteren zur Anreicherung solcher Nebenprodukte im Reaktor kommen.

Aus den vorgenannten Gründen wäre es zweckmäßig, die im Entspannungsgefäß abgeschiedene flüssige Phase vor Rückführung in den Hydroformylierungsreaktor zur Abtrennung von Restmengen an Aldehyd oder Olefin zu destillieren. Eine solche Verfahrensweise ist jedoch unwirtschaftlich, da bei der Destillation der flüssigen Phase aus dem Entspannungsgefäß diese stark aufgeheizt werden muß. Vor der Rückführung in den Hydroformylierungsreaktor müßte die so aufgeheizte flüssige Phase wieder abgekühlt werden, da andernfalls durch die im Hydroformylierungsreaktor freigesetzte Reaktionswärme die Temperatur im Hydroformylierungsreaktor auf zu hohe Werte ansteigen würde. Somit wäre ein solches Verfahren von der Energiebilanz her betrachtet sehr ungünstig.

Im Stand der Technik (EP-A 404 193, EP-A 484 976) wird die im Entspannungsbehälter abgetrennte flüssige Phase als Absorptionsmittel für diejenigen nicht umgesetzten Olefine verwendet, welche im Entspannungsbehälter in die Gasphase übergehen. Hierzu wird die Gasphase in einer Absorptionskolonne durch die gekühlte oder maximal auf derselben Temperatur wie im Entspannungsbehälter befindliche Flüssigphase geleitet und die in diesem Gasstrom enthaltenen Olefine ganz oder teilweise in dieser Flüssigphase absorbiert. Die so mit den Olefinen beladene Flüssigphase wird dann entweder wieder direkt in den Hydroformylierungsreaktor zurückgeleitet oder in einer separaten Desorptionsvorrichtung von den Olefinen befreit. Diese Verfahren sind aus den zuvor genannten Gründen insbesondere dann nachteilig, wenn als Ausgangsmaterial ein Olefingemisch aus isomeren Olefinen eingesetzt wird.

Der vorliegenden Erfindung lag deshalb die Aufgabe zugrunde ein wirtschaftliches Verfahren zur Hydroformylierung von Olefinen, insbesondere von den Olefingemischen, wie sie beispielsweise als Raffinat II nach Abtrennung darin enthaltenen 1,3-Butadiens und Isobutens aus Steamcrackern in der Technik erhalten werden, zu finden, bei dem die obengenannten Nachteile bei der weiteren Aufarbeitung des Flüssigaustrags aus dem Hydroformylierungsreaktor vermieden werden.

Dementsprechend wurde ein Verfahren zur Aufarbeitung eines im wesentlichen flüssigen, Aldehyde und nicht umgesetzte Olefine enthaltenden Austrags aus einem Hydroformylierungsreaktor aus der mit Hilfe eines homogen im Reaktionsmedium gelösten, phosphorhaltigen Rhodium-Katalysators katalysierten Niederdruckhydroformylierung bei 50 bis 150°C und 2 bis 30 bar von C₂- bis C₂₀-Olefinen oder Olefingemischen, die verschiedene Isomere der betreffenden Olefine enthalten, gefunden, das dadurch gekennzeichnet ist, daß man
a) den flüssige und gasförmige Bestandteile enthaltenden Hydroformylierungsaustrag, der außer dem Katalysator, im wesentlichen das Hydroformylierungsprodukt, höher als das Hydroformylierungsprodukt siedende Nebenprodukte, nicht umgesetztes Olefin und gesättigte Kohlenwasserstoffe und nicht umgesetztes Synthesegas enthält, in ein Entspannungsgefäß entspannt,
b) bei der Entspannung den Druck und die Temperatur soweit absenkt, daß eine im wesentlichen den Katalysator, höher als das Hydroformylierungsprodukt siedende Nebenprodukte und Restmengen an Hydroformylierungsprodukt und nicht umgesetztem Olefin enthaltende flüssige Phase und eine im wesentlichen das Hydroformylierungsprodukt, nicht umgesetztes Olefin und gesättigte Kohlenwasserstoffe und nicht umgesetztes Synthesegas enthaltende Gasphase entsteht,
c) aus der so erhaltenen flüssigen Phase einen flüssigen Strom und der so erhaltenen Gasphase einen gasförmigen Strom abzieht,
d) den flüssigen Strom anschließend auf eine um 10 bis 80°C höhere Temperatur als die im Entspannungsgefäß herrschende Temperatur erhitzt,
e) den erhitzten flüssigen Strom in flüssiger Form in den Kopfteil oder den oberen Teil einer Kolonne leitet,
f) im Sumpf oder im unteren Teil dieser Kolonne den aus dem Entspannungsgefäß abgezogenen gasförmigen Strom einleitet und dem im Kopfteil oder oberen Teil dieser Kolonne eingeführten flüssigen Strom entgegenführt,
g) am Kopf der Kolonne einen mit Olefin und Hydroformylierungsprodukt angereicherten gasförmigen Strom abzieht und der weiteren Aufarbeitung zuführt,
h) am Sumpf dieser Kolonne einen flüssigen Strom abzieht, der weniger Hydroformylierungsprodukt und Olefin als der im Kopfteil oder oberen Teil der Kolonne aufgegebene flüssige Strom enthält, und
i) diesen flüssigen Strom ganz oder teilweise wieder in den Hydroformylierungsreaktor zurückgeführt.

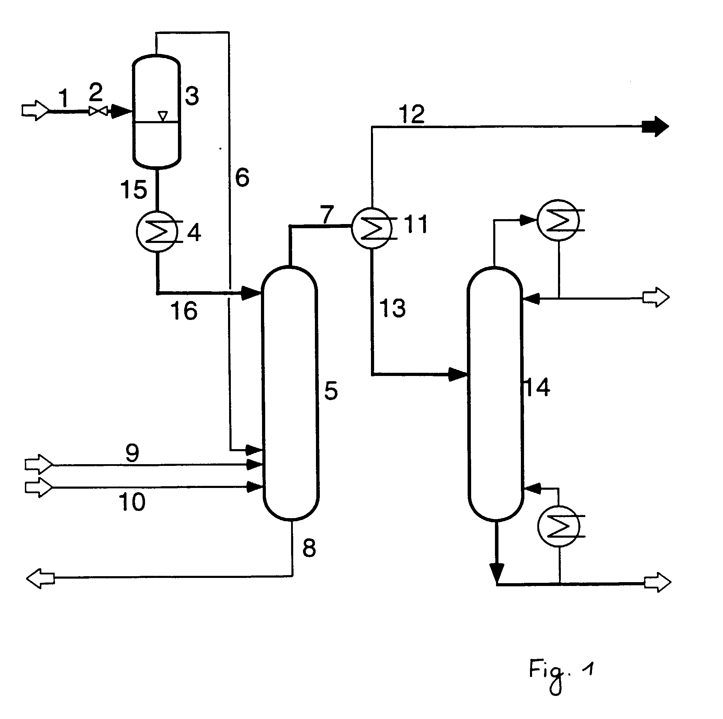

Das erfindungsgemäße Verfahren wird im Folgenden unter Zuhilfenahme der beigefügten Zeichnung unter Verwendung der darin angegebenen Bezugszeichen erläutert. Die Zeichnung ist ein allein der Erläuterung des erfindungsgemäßen Verfahrens dienendes, rein schematisches Verfahrensfließbild, in dem, aus Gründen der Übersichtlichkeit, nur die für die Erläuterung des Verfahrens notwendigen Vorrichtungen eingezeichnet sind, wohingegen andere für die Durchführung des Verfahrens notwendige und selbstverständliche Vorrichtungen, wie Pumpen, zusätzliche Ventile, Relais usw. in der Zeichnung weggelassen wurden.

Im erfindungsgemäßen Verfahren wird der im wesentlichen flüssige Austrag aus dem Hydroformylierungsreaktor, der im allgemeinen eine Temperatur von 50 bis 150°C, vorzugsweise 80 bis 110°C, hat und unter einem Druck von im allgemeinen 2 bis 30 bar, vorzugsweise 10 bis 25 bar, steht, über Leitung 1 mittels eines Ventils 2 in ein Entspannungsgefäß 3 entspannt.

Bei der angegebenen Temperatur und dem angegebenen Druck ist das im Überschuß zur Hydroformylierung eingesetzte Synthesegas - ein Kohlenmonoxid/Wasserstoff-Gemisch mit einem CO/H₂-Molverhältnis von im allgemeinen 20/80 bis 80/20, vorzugsweise von 40/60 bis 60/40 - gasförmig und als solches größtenteils in Form feiner Gasblasen im ansonsten flüssigen Austrag aus der Hydroformylierungsreaktion suspendiert. Ein geringerer Teil des nicht umgesetzten Synthesegases ist unter diesen Bedingungen im flüssigen Austrag gelöst.

Der flüssige Teil des Austrags aus der Hydroformylierungsreaktion enthält als wesentliche Bestandteile den Rhodium-Katalysator, das Hydroformylierungsprodukt, also den oder die aus dem eingesetzten Olefin oder Olefingemisch erzeugten Aldehyde, höher als das Hydroformylierungsprodukt siedende Kondensationsprodukte dieser Aldehyde, wie sie als Nebenprodukt im Zuge der Hydroformylierung entstehen und deren Zusammensetzung und Bildungsweise beispielhaft für Kondensationsprodukte des Butyraldehyds in US-A 4 158 830 erläutert wird, oder wie sie von vornherein dem Hydroformylierungsreaktor als Lösungsmittel für die Hydroformylierungsreaktion zugeführt werden, nicht umgesetztes Olefin oder nicht umgesetzte Olefine und gegebenenfalls, nicht umgesetzte, weil unreaktive, gesättigte Kohlenwasserstoffe.

Die Kohlenwasserstoffe können mit dem zur Hydroformylierung verwendeten Ausgangsmaterial in den Hydroformylierungsreaktor eingeschleppt worden sein, teilweise können sie in einer simultan zur Hydroformylierung ablaufenden Nebenreaktion, nämlich der Hydrierung der Olefine, im Hydroformylierungsreaktor gebildet worden sein.

Der im Hydroformylierungsaustrag enthaltene Rhodium-Katalysator ist ein homogen im Reaktionsmedium der Hydroformylierungsreaktion löslicher Komplex mit einer oder mehreren Organophosphorverbindung(en) als Liganden. Beispielhaft für solche Liganden seien Phosphin-Liganden aus der Klasse der Triarylphosphine, C₁-C₆-Alkyldiarylphosphine oder Arylalkyldiphosphine, wie Cyclohexyldiphenylphosphin, Hexyldiphenylphosphin, Tritolylphosphin, Tetraphenyldiphosphinomethan, 1,2-Bis(diphenylphosphino)ethan, 1,3-Bis(diphenylphosphino)propan, 1,4-Bis(diphenylphosphino)butan, insbesondere Triphenylphosphin, sowie die in EP-A 279 018, EP-A 311 619, WO 90/06810 und EP-A 71281 beschriebenen Bisphosphin-Liganden, oder sterisch gehinderte Phosphit-Liganden, wie die in US-A 4 668 651, US-A 4 748 261, US-A 4 769 498, US-A 4 774 361, US-A 4 835 299, US-A 4 885 401, US-A 5 059 710, US-A 5 113 022, US-A 5 179 055, US-A 5 260 491, US-A 5 264 616, US-A 5 288 918, US-A 5 360 938, EP-A 472 071 und EP-A 518 241 beschriebenen, chelatisierenden Phosphitliganden sowie mit jeweils 1 oder 2 Isopropyl- und/oder tert. Butylgruppen an den Phenylringen, vorzugsweise in ortho-Position zur Phosphitestergruppierung, substituierte Triphenylphosphite genannt. Da die vorgenannten Liganden bezüglich des Rhodiums im allgemeinen in einem 10 bis 1000-fachen, vorzugsweise 50 bis 500-fachen molaren Überschuß eingesetzt werden und ein solcher Überschuß des Liganden bezüglich des Rhodiums für die Aktivität, Selektivität und Stabilität des Rhodium-Katalysators von Bedeutung ist, wird für die Zwecke dieser Anmeldung aus Gründen der Vereinfachung die Mischung aus dem Rhodium-Komplex mit dem Liganden und dem freien Liganden als "Rhodium-Katalysator" oder "Katalysator" bezeichnet.

Die voranstehenden Darlegungen zum Hydroformylierungsverfahren und dem dazu verwendeten Rhodium-Katalysator, dienen dazu, das erfindungsgemäße Verfahren erläuternd in seinen technischen Gesamtzusammenhang zu stellen. Es sei an dieser Stelle betont, daß die dem erfindungsgemäßen Verfahren vorausgehende Hydroformylierung nach an sich bekannten und gebräuchlichen Hydroformylierungsverfahren mit Flüssigaustrag des Standes der Technik, beispielsweise nach den Verfahren gemäß US-A 4 148 830, EP-A 16 286, EP-A 188 246 oder EP-A 423 769 durchgeführt werden kann und in ihrer individuellen Ausgestaltung nicht Gegenstand der vorliegenden Erfindung ist.

Durch die Entspannung des im wesentlichen flüssigen Hydroformylierungsaustrags ins Entspannungsgefäß 3 wird die Trennung des im wesentlichen flüssigen Hydroformylierungsaustrags in eine im wesentlichen den Katalysator, höher als das Hydroformylierungsprodukt siedende Nebenprodukte der Hydroformylierungsreaktion, Restmengen an Olefin und Hydroformylierungsprodukt und falls ein zusätzliches hochsiedendes Lösungsmittel bei der Hydroformylierung verwendet wurde, dieses Lösungsmittel enthaltende flüssige Phase und in eine im wesentlichen den Hauptteil des Hydroformylierungsprodukts, den Hauptteil des nicht umgesetzten Olefins, gesättigte Kohlenwasserstoffe und nicht umgesetztes Synthesegas enthaltende Gasphase bewirkt. Da die je nach dem eingesetzten olefinischen Ausgangsmaterial erzeugten Hydroformylierungsprodukte, Aldehyde oder Gemische aus geradkettigen (n) oder verzweigten (iso) Aldehyden, einen unterschiedlichen Dampfdruck haben, versteht es sich für den Fachmann von selbst, daß der Druck und die Temperatur im Entspannungsgefäß unter Berücksichtigung des Dampfdrucks des jeweils vorliegenden Hydroformylierungsproduktes einzustellen sind, so daß der Hauptteil des Hydroformylierungsproduktes bei der Entspannung des flüssigen Hydroformylierungsaustrags ins Entspannungsgefäß aus der flüssigen Phase in die Gasphase übergeht.

Im allgemeinen werden der Druck und die Temperatur im Entspannungsgefäß so eingestellt, daß die im Entspannungsgefäß abgeschiedene flüssige Phase im allgemeinen nicht mehr als 10 bis 30 Gew.% Hydroformylierungsprodukt und im allgemeinen nicht mehr als 0,1 bis 10 Gew.-% Olefin oder Olefine enthält. Ein vollständiger Übergang des Hydroformylierungsprodukts und der nicht umgesetzten Olefine in die Gasphase im Entspannungsgefäß ist im allgemeinen mit wirtschaftlich vertretbarem Aufwand nicht durchführbar. Um eine solche Verteilung des Hydroformylierungsprodukts und der nicht umgesetzten Olefine zwischen der Gasphase und der flüssigen Phase im Entspannungsgefäß zu erzielen, ist bei der Einstellung der Druck- und Temperaturverhältnisse im Entspannungsgefäß der Dampfdruck des betreffenden Hydroformylierungsprodukts zu berücksichtigen: Beispielsweise wird bei der Aufarbeitung des flüssigen Austrags aus der Hydroformylierung von Raffinat II - einem Gemisch aus 1-Buten, 2-Butenen und Butanen, wie es nach Entfernung des darin enthaltenen 1,3-Butadiens und Isobutens erhalten wird - der neben den nicht umgesetzten Olefinen und Kohlenwasserstoffen n-Valeraldehyd und Isovaleraldehyd als Hydroformylierungsprodukt enthält, der Hydroformylierungsaustrag in das Entspannungsgemäß auf einen Druck von im allgemeinen 1 bis 5 bar, vorzugsweise 1 bis 3 bar, entspannt, wobei sich im Entspannungsgefäß, durch die Druckabsenkung und Verdampfung der betreffenden Komponenten eine Temperatur von im allgemeinen 50 bis 150°C, vorzugsweise 70 bis 90°C, einstellt.

Die im Entspannungsgefäß 3 abgeschiedene flüssige Phase wird als flüssiger Strom aus dem Entspannungsgefäß über Leitung 15 abgezogen und dieser beispielsweise mittels eines Durchlauferhitzers oder Wärmetauschers 4 auf eine Temperatur erhitzt, die im allgemeinen um 10 bis 80°C, vorzugsweise 20 bis 30°C, über der Temperatur der flüssigen Phase im Entspannungsgefäß 3 liegt. Grundsätzlich ist es auch möglich, die im Entspannungsgefäß abgeschiedene flüssige Phase durch eine im oder am Entspannungsgefäß installierte Heizvorrichtung auf eine um die oben angegebene Temperaturdifferenz höhere Temperatur als die Temperatur zu erhitzen, bei der die flüssige Phase ohne Beheizung des Entspannungsgefäßes im Entspannungsgefäß abgeschieden würde. Vorteilhaft wird aber der aus dem Entspannungsgefäß abgezogene flüssige Strom auf die gewünschte Temperatur erhitzt.

Der so aufgeheizte, flüssige Strom aus dem Entspannungsbehälter wird über Leitung 16 dem Kopfteil oder oberen Teil einer Kolonne 5 zugeführt, die vorteilhaft mit Füllkörpern, wie Raschig-Ringen, Spiralen oder Sattelkörpern, oder Packungen oder Einbauten, wie Rieselböden, zwecks Schaffung einer großen Oberfläche in der Kolonne bestückt ist, und im Gegenstrom dem im unteren Teil der Kolonne 5 über Leitung 6 eingeführten, aus dem oberen Teil des Entspannungsgefäßes 3 abgezogenen Gasstrom, bestehend aus der im Entspannungsgefäß gebildeten Gasphase, entgegengeführt. Beim innigen Kontakt des Gasstroms mit dem aufgeheizten flüssigen Strom, begünstigt durch die in der Kolonne vorliegende große Oberfläche, werden die im flüssigen Strom vorhandenen Restmengen an Hydroformylierungsprodukt und nicht umgesetztem Olefin in den Gasstrom transferiert, so daß der am Kopf der Kolonne 5 über Leitung 7 abgeführte Gasstrom im Vergleich zu dem am unteren Ende der Kolonne eingeführten Gasstrom an Hydroformylierungsprodukt und nicht umgesetztem Olefin angereichert, hingegen der am Sumpf die Kolonne 5 über Leitung 8 verlassende flüssige Strom, verglichen mit dem im Kopfteil oder oberen Teil der Kolonne 5 aufgegebenen flüssigen Strom, weniger Hydroformylierungsprodukt und nicht umgesetztes Olefin enthält.

Dieses Ergebnis ist sehr überraschend und unerwartet, da der im unteren Teil der Kolonne eingeführte Gasstrom bereits den größten Teil des im flüssigen Hydroformylierungsaustrag enthaltenen Hydroformylierungsprodukts und nicht umgesetzten Olefins enthält, nämlich denjenigen, der im Entspannungsgefäß in die Gasphase überführt worden ist. Aufgrund des hohen Gehalts an Hydroformylierungsprodukt und nicht umgesetztem Olefin in dem am unteren Ende der Kolonne 5 eingeführten Gasstrom und dem geringen Gehalt an Hydroformylierungsprodukt und nicht umgesetztem Olefin in dem im Kopfteil oder oberen Teil der Kolonne 5 aufgegebenen flüssigen Strom, war zu erwarten, daß das im Gasstrom in hoher Konzentration vorliegende Hydroformylierungsprodukt und nicht umgesetzte Olefin, vom flüssigen Strom unter Ausbildung eines Gleichgewichts absorbiert wird.

Der die Kolonne 5 am Sumpf über Leitung 8 verlassende, an Hydroformylierungsprodukt und nicht umgesetztem Olefin abgereicherte Flüssigkeitsstrom, der im wesentlichen aus dem Katalysator und höher als das Hydroformylierungsprodukt siedenden Nebenprodukten der Hydroformylierungsreaktion und gegebenenfalls ein zusätzlich zur Hydroformylierung verwendetes, hochsiedendes Lösungsmittel enthalten kann, wird ganz oder teilweise wieder in den Hydroformylierungsreaktor (nicht in der Zeichnung eingezeichnet) zurückgeführt. Zweckmäßigerweise wird in der Regel aus diesem flüssigen Rückführstrom, kontinuierlich oder diskontinuierlich, ein Teilstrom zwecks Ausschleusung von verbrauchtem Katalysator abgezweigt (nicht in der Zeichnung eingezeichnet). Der Abreicherungsfaktor, also das Verhältnis aus der Konzentration des Hydroformylierungsprodukts in dem der Kolonne 5 aufgegebenen flüssigen Strom zur Konzentration des Hydroformylierungsprodukts in dem am Sumpf der Kolonne 5 abgezogenen flüssigen Strom beträgt im allgemeinen 1,01 bis 3.

Der am Kopf der Kolonne 5 über Leitung 7 abgezogene, mit dem Hydroformylierungsprodukt und nicht umgesetztem Olefin angereicherte Gasstrom, der als zusätzliche nennenswerte Bestandteile gesättigte Kohlenwasserstoffe und nicht umgesetztes Synthesegas enthält, wird zweckmäßigerweise zur weiteren Aufarbeitung einem Kondensator 11 zugeführt, in dem seine höhersiedenen Bestandteile - das Hydroformylierungsprodukt, nicht umgesetztes Olefin, gesättigte Kohlenwasserstoffe - durch Kondensation vom nicht umgesetzten Synthesegas abgetrennt werden.

Das so abgetrennte, über Leitung 12 abgeführte, nicht umgesetzte Synthesegas kann gewünschtenfalls, nach Kompression auf den Druck der Hydroformylierungsreaktion, wieder in den Hydroformylierungsreaktor zurückgeführt werden. Es kann gewünschtenfalls zusätzlich frisches Synthesegas oder das über Leitung 12 abgeführte, nicht umgesetzte Synthesegas über Leitung 9 bzw. 10 in den unteren Teil der Kolonne eingeleitet werden, bevor dieses vollständig oder als Teilstrom, nach Abtrennung der kondensierbaren Bestandteile, dem Hydroformylierungsreaktor zugeführt wird. Eine solche zusätzliche Maßnahme ist für den erfolgreichen Betrieb des erfindungsgemäßen Verfahrens aber nicht notwendig.

Die im Kondensator 11 abgeschiedenen kondensierbaren Bestandteile des aus der Kolonne 5 über Leitung 7 abgeführten Gasstroms - im wesentlichen aus dem Hydroformylierungsprodukt, nicht umgesetztem Olefin und gesättigen Kohlenwasserstoffen zusammengesetzt-werden über Leitung 13 einer in der Zeichnung nur sinnbildlich dargestellten Destillationsanlage 14, die aus mehreren Destillationsanlagen bestehen kann, eingeleitet und in seine einzelnen Bestandteile zerlegt, die anschließend der weiteren Verarbeitung zu anderen Wertprodukten zugeführt werden können. Die Durchführung dieser Destillation kann nach an sich herkömmlichen Verfahren des Standes der Technik erfolgen und ist in ihrer individuellen Ausgestaltung nicht Gegenstand des erfindungsgemäßen Verfahrens.

Das erfindungsgemäße Aufarbeitungsverfahren eignet sich grundsätzlich für die Aufarbeitung von Flüssigausträgen aus der Rhodium-katalysierten Hydroformylierung von Olefinen, die aufgrund ihrer Stoffeigenschaften und aufgrund der Stoffeigenschaften der daraus gebildeten Hydroformylierungsprodukte für den Einsatz in einem Hydroformylierungsverfahren mit flüssigem Austrag der Hydroformylierungsmischung geeignet sind. Im allgemeinen sind dies C₂- bis C₂₀-Olefine. Vorzugsweise wird das erfindungsgemäße Aufarbeitungsverfahren bei der Aufarbeitung flüssiger Hydroformylierungsausträge aus der Hydroformylierung von C₂- bis C₁₀-Olefinen und besonders bevorzugt von C₂- bis C₅-Olefinen, vorzugsweise Monoolefinen, angewandt. Die eingesetzten Olefine können unsubstituiert sein oder mit 1 bis 2, vorzugsweise einem unter den Hydroformylierungsbedingungen inerten Substituenten, beispielsweise einer Estergruppe, Nitrilgruppe, Alkoxygruppe oder Hydroxygruppe substituiert sein.

Wird als Rhodium-Katalysator für die Hydroformylierung ein Komplex des Rhodiums mit einem Phosphin-Liganden eingesetzt, wird im allgemeinen unter den bereits erwähnten Bedingungen der Hydroformylierungsreaktion praktisch nur das 1-Buten zu n- und iso-Valeraldehyd hydroformyliert. Wird hingegen im Hydroformylierungsverfahren als Rhodium-Katalysator ein Komplex des Rhodiums mit einem sterisch gehinderten Phosphit-Liganden verwendet, wird das 2-Buten im Hydroformylierungsreaktor in situ zu 1-Buten isomerisiert, welches dann zu n- und iso-Valeraldehyd hydroformyliert wird.

Der flüssige Austrag aus dem Hydroformylierungsreaktor wird dann wie angegeben in das Entspannungsgefäß 3 entspannt, wobei sich eine flüssige Phase, die als wesentliche Bestandteile den Rhodium-Katalysator, höhersiedende Kondensationsprodukte des n- und iso-Valeraldehyds, gegebenenfalls ein weiteres Lösungsmittel, sowie Restmengen an n- und iso-Valeraldehyd und Restmengen nicht umgesetzter Butene, enthält und eine Gasphase, die als wesentliche Bestandteile den Hauptteil des bei der Hydroformylierung gebildeten n- und iso-Valeraldehyds, den Hauptteil der nicht umgesetzten Butene, nämlich das im Raffinat II in Spuren noch enthaltene Isobuten, je nach Art des in der Hydroformylierungsreaktion verwendeten Rhodium-Katalysators gegebenenfalls das 2-Buten, und allenfalls geringe Mengen 1-Buten oder gegebenenfalls, abhängig vom Rhodium-Katalysator geringe Mengen 2-Buten, sowie verschiedenerlei Butan-Isomere und nicht-umgesetztes Synthesegas enthält, bilden.

Aus der im Entspannungsgefäß 3 abgeschiedenen flüssigen Phase wird kontinuierlich über Leitung 15 ein flüssiger Strom abgezogen, dessen Temperatur in einem Wärmetauscher 4 vorzugsweise um 20 bis 80°C erhöht wird und der nach Passieren des Wärmetauschers 4 über Leitung 16 in den Kopfteil oder oberen Teil der Kolonne 5 aufgegeben wird. Aus der im Entspannungsbehälter 3 abgeschiedenen Gasphase wird ein kontinuierlicher Gasstrom über Leitung 6 abgezogen, der vorzugsweise ohne weitere Aufheizung in den Sumpf oder den unteren Teil der Kolonne 5 gepumpt und dem am Kopfteil oder oberen Teil der Kolonne 5 aufgegebenen, flüssigen Strom entgegengeführt wird. Am Sumpf der Kolonne wird ein an Valeraldehyden abgereicherter oder freier flüssiger Strom über Leitung 8 abgezogen, der den Rhodium-Katalysator und die höher siedenden Kondensationsprodukte der isomeren Valeraldehyde und gegebenenfalls ein weiteres hochsiedendes Lösungsmittel enthält, und vollständig oder nach Ausschleusung eines Teilstroms zur Aufarbeitung verbrauchten Rhodium-Katalysators in den Hydroformylierungsreaktor zurückgeführt wird. Am Kopf der Kolonne 5 wird ein mit den Valeraldehyden und nicht umgesetzten Olefinen angereicherter Gasstrom abgeführt und über Leitung 7 dem Kondensator 11 zugeführt, worin seine kondensierbaren Bestandteile, im wesentlichen n- und iso-Valeraldehyd, Isobuten, Butane, und je nach dem in der Hydroformylierung verwendeten Katalysator größere oder kleinere Mengen 2-Buten sowie Restmengen von 1-Buten, kondensiert und über Leitung 13 der destillativen Aufarbeitung zugeführt werden. Das im Kondensator 11 nicht kondensierbare, nicht umgesetzte Synthesegas wird entweder vollständig oder teilweise nach Kompression über Leitung 12 in den Hydroformylierungsreaktor zurückgeführt. Bei der destillativen Aufarbeitung des im Kondensator 11 erhaltenen flüssigen Kondensats werden zweckmäßigerweise zunächst die leichtflüchtigen Butene und Butane vom Valeraldehydgemisch abgetrennt und anschließend in die einzelnen Bestandteile Isobuten, gegebenenfalls 2-Butene, Buten-1 und Butane aufgetrennt. Das 2-Buten und die Restmengen an 1-Buten können zur Butendimerisierung und die Butane können als Einsatzstoff in einem Steamcracker verwendet werden. Das erhaltene n-/iso-Valeraldehydgemisch kann gewünschtenfalls in die einzelnen Isomere aufgetrennt werden oder als solches zur Herstellung des Weichmacheralkohols 2-Propylheptanol eingesetzt werden. Gewünschtenfalls können die bei der Destillation abgetrennten, gasförmigen Butene, also das Abgas des Verfahrens, über Leitung 10 vollständig oder als Teilstrom in die Kolonne 5 zurückgeführt werden.

Das erfindungsgemäße Verfahren ermöglicht eine bessere und vollständigere Abtrennung der im flüssigen Hydroformylierungsaustrag enthaltenen Aldehyde von den anderen Bestandteilen auf wirtschaftlichere Weise als der bisherige Stand der Technik. Insbesondere ermöglicht das erfindungsgemäße Verfahren die Gewinnung der Hydroformylierungsprodukte aus dem flüssigen Hydroformylierungsaustrag mit wesentlich niedrigerem Energieaufwand als bekannte Verfahren. Infolge der milden Bedingungen bei der erfindungsgemäßen Aufarbeitung des flüssigen Hydroformylierungsaustrags wird der Rhodium-Katalysator geschont und es bilden sich bei der Aufarbeitung weniger Nebenprodukte aus dem Hydroformylierungsprodukt, wodurch die Gesamtselektivität des Verfahrens und damit dessen Wirtschaftlichkeit erhöht werden.

## Patentansprüche

1. Verfahren zur Aufarbeitung eines im wesentlichen flüssigen, Aldehyde und nicht umgesetzte Olefine enthaltenden Austrags aus einem Hydroformylierungsreaktor aus der mit Hilfe eines homogen im Reaktionsmedium gelösten phosphorhaltigen Rhodium-Katalysators katalysierten Niederdruckhydroformylierung bei 50 bis 150°C und 2 bis 30 bar von C₂- bis C20-Olefinen oder Olefingemischen, die verschiedene Isomere der betreffenden Olefine enthalten, dadurch gekennzeichnet, daß man
a. den flüssige und gasförmige Bestandteile enthaltenden Hydroformylierungsaustrag, der außer dem Katalysator im wesentlichen das Hydroformylierungsprodukt, höher als das Hydroformylierungsprodukt siedende Nebenprodukte, nicht umgesetztes Olefin und gesättigte Kohlenwasserstoffe und nicht umgesetztes Synthesegas enthält, in ein Entspannungsgefäß entspannt,
b. bei der Entspannung den Druck und die Temperatur soweit absenkt, daß eine im wesentlichen den Katalysator, höher als das Hydroformylierungsprodukt siedende Nebenprodukte und Restmengen an Hydroformylierungsprodukt und nicht umgesetztem Olefin enthaltende flüssige Phase und eine im wesentlichen das Hydroformylierungsprodukt, nicht umgesetztes Olefin und gesättigte Kohlenwasserstoffe und nicht umgesetztes Synthesegas enthaltende Gasphase entsteht,
c. aus der so erhaltenen flüssigen Phase einen flüssigen Strom und der so erhaltenen Gasphase einen gasförmigen Strom abzieht,
d. den flüssigen Strom anschließend auf eine um 10 bis 80°C höhere Temperatur als die im Entspannungsgefäß herrschende Temperatur erhitzt,
e. den erhitzten flüssigen Strom in flüssiger Form in den Kopfteil oder den oberen Teil einer Kolonne leitet,
f. im Sumpf oder im unteren Teil dieser Kolonne den aus dem Entspannungsgefäß abgezogenen gasförmigen Strom einleitet und dem im Kopfteil oder oberen Teil dieser Kolonne eingeführten flüssigen Strom entgegenführt,
g. am Kopf der Kolonne einen mit Olefin und Hydroformylierungsprodukt angereicherten gasförmigen Strom abzieht und der weiteren Aufarbeitung zuführt,
h. am Sumpf dieser Kolonne einen flüssigen Strom abzieht, der weniger Hydroformylierungsprodukt und Olefin als der im Kopfteil oder oberen Teil der Kolonne aufgegebene flüssige Strom enthält, und
i. diesen flüssigen Strom ganz oder teilweise wieder in den Hydroformylierungsreaktor zurückführt.

2. Verfahren nach Anspruch 1, daß man als Olefin Ethylen einsetzt und Propionaldehyd erzeugt.

3. Verfahren nach Anspruch 1, daß man als Olefin Propylen einsetzt und n- und iso-Butanal erzeugt.

4. Verfahren nach Anspruch 1, daß man als Olefin 1-Buten oder ein 1-Buten enthaltendes Kohlenwasserstoffgemisch einsetzt und n- und iso-Valeraldehyd erzeugt.

## Claims

1. A process for working up an essentially liquid product comprising aldehydes and unreacted olefins from a hydroformylation reactor in which the low-pressure hydroformylation of C2-C20-olefins or olefin mixtures comprising various isomers of these olefins is carried out at from 50 to 150°C and from 2 to 30 bar in the presence of a phosphorus-containing rhodium catalyst homogeneously dissolved in the reaction medium, which comprises
a. depressurizing the hydroformylation product which comprises liquid and gaseous constituents and consists essentially of the hydroformylation product, by-products having boiling points higher than that of the hydroformylation product, unreacted olefin and saturated hydrocarbons and unreacted synthesis gas as well as the catalyst into an expansion vessel,
b. lowering the pressure and the temperature in the depressurization sufficiently for a liquid phase comprising essentially the catalyst, by-products having higher boiling points than that of the hydroformylation product and residual amounts of hydroformylation product and unreacted olefin and a gas phase comprising essentially the hydroformylation product, unreacted olefin and saturated hydrocarbons and unreacted synthesis gas to be formed,
c. taking off a liquid stream from the liquid phase obtained in this way and taking off a gaseous stream from the gas phase obtained in this way,
d. subsequently heating the liquid stream to a temperature which is from 10 to 80°C higher than the temperature prevailing in the expansion vessel,
e. conveying the heated liquid stream in liquid form into the top or the upper part of a column,
f. introducing the gaseous stream taken off from the expansion vessel into the bottom or the lower part of this column and passing it through the column in countercurrent to the liquid stream introduced into the top or upper part of this column,
g. taking off a gaseous stream enriched with olefin and hydroformylation product at the top of the column and passing it to further work-up,
h. at the bottom of this column, taking off a liquid stream which contains less hydroformylation product and olefin than the liquid stream introduced into the top or upper part of the column, and
i. recirculating all or part of this liquid stream to the hydroformylation reactor.

2. A process as claimed in claim 1, wherein ethylene is used as olefin and propionaldehyde is produced.

3. A process as claimed in claim 1, wherein propylene is used as olefin and n- and iso-butanal are produced.

4. A process as claimed in claim 1, wherein 1-butene or a hydrocarbon mixture containing 1-butene is used as olefin and n- and iso-valeraldehyde are produced.

## Revendications

1. Procédé de recyclage d'un produit de décharge essentiellement liquide provenant d'un réacteur d'hydroformylation et contenant des aldéhydes et des oléfines n'ayant pas réagi, à partir de l'hydroformylation sous basse pression, catalysée à l'aide d'un catalyseur au rhodium contenant du phosphore dissous de façon homogène dans le milieu réactionnel, à 50-150°C et sous 2-30 bar, d'oléfines en C₂-C₂₀ ou de mélanges d'oléfines contenant différents isomères des oléfines en question,
caractérisé en ce que
a. l'on détend, dans un récipient de détente, les constituants liquides et gazeux contenus dans le produit de décharge de l'hydroformylation, qui contient en plus du catalyseur, essentiellement le produit de l'hydroformylation, les produits secondaires bouillant plus haut que le produit de l'hydroformylation, l'oléfine n'ayant pas réagi et les hydrocarbures saturés ainsi que le gaz de synthèse n'ayant pas réagi,
b. on diminue la température et la pression, lors de la détente, jusqu'à ce qu'il en résulte une phase liquide contenant essentiellement le catalyseur, les produits secondaires bouillant plus haut que le produit de l'hydroformylation, et des quantités résiduelles du produit d'hydroformylation et l'oléfine n'ayant pas réagi, et une phase gazeuse contenant essentiellement le produit d'hydroformylation, l'oléfine n'ayant pas réagi et les hydrocarbures saturés ainsi que le gaz de synthèse n'ayant pas réagi,
c. on extrait de la phase liquide ainsi obtenue un courant liquide et de la phase gazeuse ainsi obtenue un courant gazeux,
d. on chauffe ensuite le courant liquide à une température supérieure de 10 à 80°C à celle régnant dans le récipient de détente,
e. on introduit le courant liquide chauffé, sous forme liquide, dans la partie de tête ou dans la partie supérieure d'une colonne,
f. on introduit en fond ou dans la partie inférieure de cette colonne le courant gazeux extrait du récipient de détente, et on le fait circuler à contre-courant du courant liquide introduit dans la partie de tête ou dans la partie supérieure de la colonne,
g. on extrait un courant gazeux enrichi en oléfine et en produit d'hydroformylation en tête de colonne et on le conduit jusqu'au recyclage suivant,
h. on extrait un courant liquide en fond de colonne, contenant moins de produit d'hydroformylation et d'oléfine que le courant liquide introduit dans la partie de tête ou la partie supérieure de la colonne, et
i. on renvoie tout ou partie de ce courant liquide dans le réacteur d'hydroformylation.

2. Procédé selon la revendication 1, caractérisé en ce que l'on utilise de l'éthylène en tant qu'oléfine et on obtient du propionaldéhyde.

3. Procédé selon la revendication 1, caractérisé en ce que l'on utilise du propylène en tant qu'oléfine et on obtient du n- et de l'iso-butanal.

4. Procédé selon la revendication 1, caractérisé en ce que l'on utilise du butène-1 ou un mélange d'hydrocarbures contenant du butène-1, en tant qu'oléfine et on obtient du n- et de l'iso-valéraldéhyde.
